(19) Europäisches Patentamt
European Patent Office
Office européen des brevets

(11) Veröffentlichungsnummer: **0 107 027**
**B1**

(12) # EUROPÄISCHE PATENTSCHRIFT

(45) Veröffentlichungstag der Patentschrift: **18.12.85**

(51) Int. Cl.⁴: **A 61 K 7/13,** C 07 C 87/58

(21) Anmeldenummer: **83109211.9**

(22) Anmeldetag: **17.09.83**

(54) **Haarfärbemittel.**

(30) Priorität: **25.09.82 DE 3235615**

(43) Veröffentlichungstag der Anmeldung: **02.05.84 Patentblatt 84/18**

(45) Bekanntmachung des Hinweises auf die Patenterteilung: **18.12.85 Patentblatt 85/51**

(84) Benannte Vertragsstaaten: **AT BE CH DE FR GB IT LI NL SE**

(56) Entgegenhaltungen:
**EP - A - 0 011 844**
**DE - A - 1 959 462**
**DE - C - 365 785**

**HELVETIA CHIMICA ACTA, Band 22, 1939, Seiten 970-978, Basel, CH FR. FICHTER u.a.: "Die Kolbe'sche Elektrosynthese mit aromatischen Säuren: Benzoesäure, Phenyl-essigsäure, beta.Phenyl-propionsäure, Phenoxy-essigsäure"**

(73) Patentinhaber: **Henkel Kommanditgesellschaft auf Aktien, Postfach 1100 Henkelstrasse 67, D-4000 Düsseldorf-Holthausen (DE)**

(72) Erfinder: **Rose, David, Dr., Holbeinweg 7, D-4010 Hilden (DE)**
Erfinder: **Lieske, Edgar, Hunsrückenstrasse 40, D-4000 Düsseldorf (DE)**

EP 0 107 027 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung sind Haarfärbemittel auf der Basis von Oxidationsfarbstoffen. Solche Haarfärbemittel enthalten Oxidationsfarbstoffvorprodukte in einem kosmetischen Träger. Als Oxidationsfarbstoffvorprodukte werden Entwicklersubstanzen und Kupplersubstanzen eingesetzt, die unter dem Einfluss von Oxidationsmitteln oder von Luftsauerstoff Farbstoffe ausbilden. Als kosmetische Träger für die Oxidationsfarbstoffvorprodukte dienen Cremes, Emulsionen, Gele, Shampoos, Schaumaerosole oder andere Zubereitungen, die für die Anwendung auf dem Haar geeignet sind.

Für das Färben von Haaren spielen die sogenannten Oxidationsfarben, die durch oxidative Kupplung einer oder mehrerer Entwicklerkomponenten untereinander oder mit einer oder mehreren Kupplerkomponenten entstehen, wegen ihrer intensiven Farben und guten Echtheitseigenschaften eine bevorzugte Rolle. Als Entwicklersubstanzen werden üblicherweise primäre aromatische Amine mit einer weiteren in Para- oder Orthoposition befindlichen freien oder substituierten Hydroxy- oder Aminogruppe, ferner Diaminopyridinderivate, heterocyclische Hydrazonderivate, 4-Aminopyrazolonderivate und Tetraaminopyrimidine eingesetzt. Als sogenannte Kupplersubstanzen werden m-Phenylendiaminderivate, Phenole, Naphthole, Resorcinderivate und Pyrazolone verwendet.

Gute Oxidationshaarfarbstoffvorprodukte müssen in erster Linie folgende Voraussetzungen erfüllen: Sie müssen bei der oxidativen Kupplung die gewünschten Farbnuancen in ausreichender Intensität ausbilden. Sie müssen ferner ein gutes Aufziehvermögen auf menschlichem Haar besitzen, ohne die Kopfhaut zu stark anzufärben, und sie sollen vor allem in toxikologischer und dermatologischer Hinsicht unbedenklich sein.

Aus der deutschen Patentschrift Nr. 365785 sind Tetraaminoderivate des Diphenyls, des Diphenylmethans und Diphenylethans als Oxidationsfarbstoffvorprodukte bekannt. Die mit diesen Substanzen erzielbaren Färbungen sind jedoch in der Reinheit und Intensität und in der Stabilität gegenüber Wärme und Kaltwell-Behandlungen des Haares nicht befriedigend. So liefern Bis-(2,4-diaminophenyl)methan und 1,2-Bis-(2',4'-diaminophenyl)ethan mit P-Toluylendiamin braungraue bis braunschwarze Nuancen, die unter dem Einfluss von Wärme oder Thioglycolat-Lösungen einen schmutzigen, unreinen Braunton annehmen.

Aus der deutschen Offenlegungsschrift Nr. 2852156 sind Bis-(2,4-diaminophenoxy)-alkane und deren Verwendung als Oxidationsfarbstoffvorprodukte bekannt. Es bestand jedoch der Wunsch, Kupplerkomponenten mit noch besseren toxikologisch-dermatologischen Eigenschaften zu schaffen.

Bei der Suche nach besseren Oxidationshaarfarbstoffvorprodukten bestand daher die Aufgabe, geeignete Kupplersubstanzen aufzufinden, die sowohl in färberischer als auch in dermatologischer Hinsicht die gestellten Aufgaben in optimaler Weise erfüllen.

Diese Aufgabe wurde erfindungsgemäss gelöst durch neue Haarfärbemittel, enthaltend Oxidationsfarbstoffvorprodukte und einen kosmetischen Träger, dadurch gekennzeichnet, dass als Oxidationsfarbstoffvorprodukte Bis-(2,4-diaminophenyl)alkane der allgemeinen Formel I:

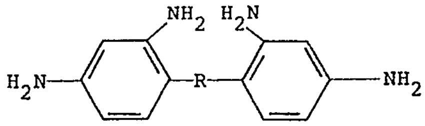

in der R eine Alkylengruppe mit drei oder vier Kohlenstoffatomen darstellt oder deren Salze als Kupplerkomponenten und die in Oxidationsfärbemitteln üblichen Entwicklersubstanzen enthalten sind. Unter den Kupplersubstanzen der allgemeinen Formel I sind das 1,3-Bis-(2',4'-diaminophenyl)propan und das 1,4-Bis-(2',4'-diaminophenyl)butan als besonders bevorzugt geeignete Kuppler zu nennen.

Die Bis-(2,4-diaminophenyl)alkane der allgemeinen Formel I, in der R eine Alkylengruppe mit drei oder vier Kohlenstoffatomen darstellt, sind bisher nicht bekannt. Ein weiterer Erfindungsgegenstand sind daher die neuen Bis-(2,4-diaminophenyl)alkane.

Die Herstellung der erfindungsgemässen Bis-(2,4-diaminophenyl)alkane erfolgt analog zu literaturbekannten Herstellungsverfahren durch katalytische Hydrierung der entsprechenden Bis-(2,4-dinitrophenyl)alkane.

Die neuen Kupplersubstanzen der allgemeinen Formel I eignen sich zwar für eine Vielzahl verschiedener Entwicklersysteme, besonders intensive und reine Grün- und Blaunuancen, werden jedoch vor allem bei Verwendung von aromatischen und heterocyclischen Diaminen als Entwicklersubstanzen erhalten. Solche bevorzugten Entwicklersysteme sind z. B. p-Phenylendiamin, p-Tolylendiamin, N-Methyl-p-phenylendiamin, N,N-Dimethyl-p-phenylendiamin, N,N-Diäthyl-2-methyl-p-phenylendiamin, N-Äthyl-N-hydroxyäthylp-phenylendiamin, Chlor-p-phenylendiamin, N,N-Bis-hydroxyäthylamino-p-phenylendiamin, Methoxy-p-phenylendiamin, 2,6-Dichlor-p-phenylendiamin, 2-Chlor-6-brom-p-phenylendiamin, 2-Chlor-6-methyl-p-phenylendiamin, 6-Methoxy-3-methyl-p-phenylendiamin, 2,5-Diaminoanisol, N-Ethyl-(N-(2-hydroxyethyl)-p-phenylendiamin, N-2-Methoxyethyl-p-phenylendiamin, N-2-Hydroxypropyl-p-phenylendiamin sowie andere Verbindungen der genannten Art, die weiterhin eine oder mehrere $NH_2$-Gruppen, NHR-Gruppen, $NR_2$-Gruppen aufweisen, wobei R einen Alkylrest mit 1-4 oder einen Hydroxyalkylrest mit 2-4 Kohlenstoffatomen darstellt, ferner Diaminopyridinderivate, N-Butyl-N-sulfobutyl-p-phenylendiamin, Tetraaminopyrimidine wie 2,4,5,6-Tetraaminopyrimidin, 4,5-Diamino-2,6-bismethylaminopyrimidin, 2,5-Diamino-4-diäthylamino-6-methylaminopyrimidin, 2,4,5-Triamino-6-dimethylaminopyrimidin, 2,4,5-Triamino-6-piperidinopyrimi-

din, 2,4,5-Triamino-6-anilinopyrimidin, 2,4,5-Triamino-6-morpholinopyrimidin, 2,4,5-Triamino-6-β-hydroxyäthylaminopyrimidin.

Die erfindungsgemässen Haarfärbemittel erzeugen auf dem Haar sehr intensive, grüne bis tiefblaue Farbnuancen von hoher Reinheit, Echtheit und Beständigkeit gegen Licht, Wärme und den Einfluss von Kaltwellbehandlungen auf Basis von Merkaptanen. Sie stellen daher eine wesentliche Bereicherung der oxidativen Haarfärbemöglichkeiten dar.

Die neuen Kupplerkomponenten können entweder als freie Basen oder in Form ihrer Salze mit anorganischen oder organischen Säuren, z. B. als Hydrochloride, Sulfate, Phosphate, Acetate, Propionate, Lactate oder Citrate in die erfindungsgemässen Haarfärbemittel eingearbeitet werden. Die erfindungsgemässen Haarfärbemittel können darüber hinaus weitere bekannte Kupplersubstanzen, z. B. Naphthole, Resorcinderivate, Pyrazolone und m-Phenylendiaminderivate enthalten. Auch direktziehende Farbstoffe, z.B. Nitrophenylendiaminderivate, können zur Modifikation der Farbnuancen zugesetzt werden.

In den erfindungsgemässen Haarfärbemitteln werden die Kupplersubstanzen im allgemeinen in etwa molaren Mengen, bezogen auf die verwendeten Entwicklersubstanzen, eingesetzt. Wenn sich auch der molare Einsatz als zweckmässig erweist, so ist es jedoch nicht nachteilig, einen gewissen Überschuss einzelner Oxidationsfarbstoffvorprodukte zum Einsatz zu bringen.

Es ist ferner nicht erforderlich, dass die Oxidationsfarbstoffvorprodukte einheitliche Substanzen darstellen, vielmehr können sowohl die Entwicklerkomponente Gemische der erfindungsgemäss zu verwendenden Entwicklerverbindungen als auch die Kupplersubstanz Gemische der erfindungsgemäss einzusetzenden Bis-(2,4-Diaminophenyl)alkane darstellen.

Die oxidative Entwicklung der Färbung kann grundsätzlich, wie bei anderen Oxidationshaarfarbstoffen auch, durch Luftsauerstoff erfolgen. Zweckmässigerweise werden jedoch chemische Oxidationsmittel eingesetzt. Als solche kommen insbesondere Wasserstoffperoxid oder dessen Anlagerungsprodukte an Harnstoff, Melamin und Natriumborat sowie Gemische aus derartigen Wasserstoffperoxidanlagerungsverbindungen mit Kaliumperoxiddisulfat in Betracht.

Die erfindungsgemäss einzusetzenden Bis-(2,4-Diaminophenyl)alkane besitzen ferner den Vorteil, dass sie bereits bei oxidativer Kupplung durch Luftsauerstoff befriedigende Färbeergebnisse liefern. Wird jedoch gleichzeitig neben der Färbung ein Aufhelleffekt am Haar gewünscht, so ist die Mitverwendung von Oxidationsmitteln erforderlich.

Zur Herstellung der erfindungsgemässen Haarfärbemittel werden die Oxidationsfarbstoff-Vorprodukte in einen geeigneten kosmetischen Träger eingearbeitet. Solche Träger sind z. B. Cremes, Emulsionen, Gele oder auch tensidhaltige, schäumende Lösungen, z. B. Shampoos oder auch Zubereitungen, die für die Anwendungen auf dem Haar geeignet sind. Übliche Bestandteile solcher kosmetischer Zubereitungen sind zum Beispiel Netz- und Emulgiermittel, wie anionische, nichtionische oder ampholytische Tenside, z.B. Fettalkoholsulfate, Alkansulfonate, α-Olefinsulfonate, Fettalkoholpolyglycoläthersulfate, Ethylenoxidanlagerungsprodukte an Fettalkohole, Fettsäuren und Alkylphenole, Sorbitanfettsäureester und Fettsäurepartialglyceride, Fettsäurealkanolamide sowie Verdickungsmittel wie z. B. Methyl- oder Hydroxyethylcellulose, Stärke, Fettalkohole, Paraffinöl, Fettsäuren, ferner Parfümöle und haarpflegende Zusätze wie z. B. wasserlösliche kationische Polymere, Proteinderivate, Pantothensäure und Cholesterin.

Die Bestandteile der kosmetischen Träger werden zur Herstellung der erfindungsgemässen Haarfärbemittel in für diese Zwecke üblichen Mengen eingesetzt; z. B. werden Emulgiermittel in Konzentrationen von 0,5 bis 30 Gew.-% und Verdickungsmittel in Konzentrationen von 0,1 bis 25 Gew.-% der Haarfärbemittel eingesetzt. Die Oxidationsfarbstoffvorprodukte werden in Mengen von 0,05 bis 5,0 Gew.-%, vorzugsweise 1 bis 3 Gew.-% des gesamten Färbemittels in den Träger eingemischt.

Die Anwendung der erfindungsgemässen Haarfärbemittel kann, unabhängig davon, ob es sich um eine Lösung, eine Emulsion, eine Creme oder ein Gel handelt, im schwach sauren, neutralen oder insbesondere alkalischen Milieu bei einem pH-Wert von 8-10 erfolgen. Die Anwendungstemperaturen bewegen sich dabei im Bereich von 15 bis 40° C. Nach einer Einwirkungsdauer von ca. 30 min wird das Haarfärbemittel vom zu färbenden Haar durch Spülen entfernt. Hernach wird das Haar mit einem milden Shampoo nachgewaschen und getrocknet. Das Nachwaschen mit einem Shampoo entfällt, wenn ein stark tensidhaltiger Träger, z. B. ein Färbeshampoo, verwendet wurde.

Die mit den erfindungsgemässen Haarfärbemitteln erzielbaren Färbungen haben gute Licht-, Wasch- und Reibechtheitseigenschaften.

Die nachfolgenden Beispiele sollen den Erfindungsgegenstand näher erläutern, ohne ihn jedoch hierauf zu beschränken.

*Beispiele*

I. *Herstellung der erfindungsgemässen Bis-(2,4-diaminophenyl)alkane*

A. *1,3-Bis-(2',4'-diaminophenyl)propantetrahydrochlorid*

*1. Stufe: 1,3-Bis-(2',4'-dinitrophenyl)propan*

5 g 1,3-Diphenylpropan wurden bei einer Temperatur von −5 bis −10° C zu 50 ml Salpetersäure mit einer Dichte von d = 1,52 g/ml zugetropft. Innerhalb von 2 h wurde die Lösung auf eine Temperatur von ca. 20° C gebracht. Das Reaktionsgemisch wurde dann auf Eiswasser gegossen, abfiltriert und der Rückstand neutral gewaschen. Zur Reinigung wurde der Rückstand mit Ethanol ausgekocht und einmal aus Essigsäure und zweimal aus Methylethylketon umkristallisiert. Die Ausbeute betrug 2,4 g. Das Produkt hatte einen Schmelzpunkt von 168-170° C.

*2. Stufe: Hydrierung*

2,3 g Bis-(2',4'-dinitrophenyl)propan wurden in 100 ml Ethanol gelöst und nach Zugabe von Palladium auf A-Kohle (5 Gew.-% Pd) in Wasserstoffatmosphäre katalytisch hydriert. Nach Beendigung der Wasserstoff-Aufnahme wurde der Katalysator abfiltriert, das Filtrat mit konzentrierter Salzsäure angesäuert und zur Trockene eingeengt. Das Produkt wurde in Form weisser Kristalle erhalten, die bei ca. 306° C unter Zersetzung schmelzen.

B. *1,4-Bis-(2',4'-diaminophenyl)butantetrahydrochlorid*

*1. Stufe: 1,4-Bis-(2',4'-dinitrophenyl)butan*

Die Herstellung erfolgt nach Fr. Fichter und H. Stenzl, «Helv. Chim. Acata», *22* (1939), S. 975.

*2. Stufe: Hydrierung*

4 g 1,4-Bis-(2',4'-dinitrophenyl)butan wurden in 100 ml Pthanol gelöst und nach Zugabe von Palladium auf A-Kohle (5 Gew.-% Pd) in Wasserstoffatmosphäre katalytisch hydriert. Nach Beendigung der Wasserstoffaufnahme wurde der Katalysator abfiltriert, das Filtrat mit konzentrierter Salzsäure angesäuert und zur Trockene eingeengt. Das Produkt wurde in Form beigefarbener Kristalle erhalten, die bei ca. 310° C unter Zersetzung schmelzen.

II. *Oxidationshaarfärbemittel-Prüfung*

Die vorgenannten, in ihrer Herstellung beschrieben neuen Bis-(2,4-diaminophenyl)alkane wurden als Kupplerkomponenten in Haarfärbe-Cremeemulsionen der folgenden Zusammensetzung eingesetzt:

| | |
|---|---|
| Fettalkohol $C_{12-18}$ | 10 g |
| Fettalkohol $C_{12-18}$-sulfat, Na-Salz | 10 g |
| Wasser | 75 g |
| Entwicklersubstanz | 0,01 mol |
| Kupplersubstanz | 0,01 mol |
| konz. Ammoniak-Lösung | bis pH = 9,5 |
| Wasser | ad 100 g |

Die Bestandteile wurden der Reihe nach miteinander vermischt. Nach Zugabe der Entwicklersubstanz und der Kupplersubstanz wurde zunächst mit konzentrierter Ammoniaklösung der pH-Wert der Emulsion auf 9,5 eingestellt, dann mit Wasser auf 100 g aufgefüllt.

Die oxidative Kupplung wurde mit 9%iger Wasserstoffperoxidlösung als Oxidationsmittel durchgeführt. Hierzu wurden 100 g der Emulsion mit 10 g Wasserstoffperoxidlösung (9%ige) versetzt und vermischt.

Die Färbecreme wurde auf standardisiertes, zu 90% ergrautes aber nicht besonders vorbehandeltes Menschenhaar aufgetragen und dort 30 min bei 35° C belassen. Nach Beendigung des Färbeprozesses wurde das Haar gespült, mit einem üblichen Haarwaschmittel ausgewaschen und anschliessend getrocknet.

Die als Entwicklersubstanzen verwendeten Stoffe waren

E 1: p-Phenylendiamin
E 2: p-Toluylendiamin
E 3: N-Methyl-p-phenylendiamin
E 4: 2-Chlor-p-phenylendiamin
E 5: 2,5-Diaminoanisol
E 6: N-Ethyl-N-(2-hydroxyethyl)-p-phenylendiamin
E 7: N-Butyl-N-sulfobutyl-p-phenylendiamin
E 8: N,N-Bis-(2-hydroxyethyl)-p-phenylendiamin
E 9: N-(2-methoxyethyl)-p-phenylendiamin
E 10: N-(2-hydroxypropyl)-p-phenylendiamin
E 11: 2,4,5,6-Tetraaminopyrimidin
E 12: p-Aminophenol

Die mit diesen Entwicklern und den Kupplern A und B erhaltenen Färbungen sind der nachstehenden Tabelle zu entnehmen. Zum Vergleich wurden als Kuppler auch
C: Bis-(2,4-diaminophenyl)methan
D: 1,2-Bis-(2',4'-diaminophenyl)ethan
eingesetzt.

| Beispiel | Entwickler | Kuppler | erhaltener Farbton |
|---|---|---|---|
| 1 | E 1 | A | schwarzblau |
| 2 | E 2 | A | tintenblau |
| 3 | E 3 | A | schwarzblau |
| 4 | E 4 | A | schwarzblau |
| 5 | E 5 | A | tintenblau |
| 6 | E 6 | A | nordischblau |
| 7 | E 7 | A | mattblau |
| 8 | E 8 | A | tintenblau |
| 9 | E 9 | A | schwarzblau |
| 10 | E 10 | A | tintenblau |
| 11 | E 11 | A | dunkelgrün |
| 12 | E 12 | A | violettbraun |
| 13 | E 2 | B | dunkelblau |
| 14 | E 2 | C | braungrau |
| 15 | E 2 | D | schwarzgrau |

## Patentansprüche

1. Haarfärbemittel, enthaltend Oxidationsfarbstoffvorprodukte in einem kosmetischen Träger, dadurch gekennzeichnet, dass als Oxidationsfarbstoffvorprodukte Bis-(2,4-diaminophenyl)alkane der allgemeinen Formel (I)

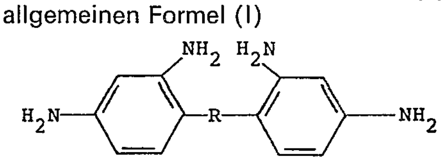

(I)

in der R eine Alkylengruppe mit drei oder vier Kohlenstoffatomen darstellt oder deren Salze als Kupplerkomponenten und die in Oxidationsfärbemitteln üblichen Entwicklersubstanzen enthalten sind.

2. Haarfärbemittel nach Anspruch 1, dadurch gekennzeichnet, dass R eine 1,3-Propylen- oder eine 1,4-Butylengruppe darstellt.

3. Haarfärbemittel nach Anspruch 1 oder 2, dadurch gekennzeichnet, dass als Entwicklersubstanzen aromatische und heterocyclische Diamine enthalten sind.

4. Haarfärbemittel nach Anspruch 1 bis 3, dadurch gekennzeichnet, dass zusätzliche weitere bekannte Kupplersubstanzen und/oder direktziehende Farbstoffe enthalten sind.

5. Haarfärbemittel nach Anspruch 1 bis 4, dadurch gekennzeichnet, dass der Gehalt an Oxidationsfarbstoffvorprodukten 0,05 bis 5,0 Gew.-% des gesamten Färbemittels ausmacht.

6. Bis-(2,4-diaminophenyl)alkane der allgemeinen Formel (I) in Anspruch 1, in der R eine Alkylengruppe mit drei oder vier Kohlenstoffatomen darstellt.

## Claims

1. Hair dyeing preparations containing oxidation dye precursors in a cosmetic carrier, characterized in that they contain bis-(2,4-diaminophenyl)alkanes corresponding to the following general formula

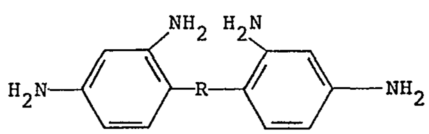

(I)

in which R is an alkylene group containing three or four carbon atoms, or salts thereof as coupler components and the usual developers for oxidation dyes as oxidation dye precursors.

2. Hair dyeing preparations as claimed in Claim 1, characterized in that R is a 1,3-propylene or 1,4-butylene group.

3. Hair dyeing preparations as claimed in Claim 1 or 2, characterized in that they contain aromatic and heterocyclic diamines as developers.

4. Hair dyeing preparations as claimed in Claims 1 to 3, characterized in that they additionally contain other known couplers and/or substantive dyes.

5. Hair dyes as claimed in Claims 1 to 4, characterized in that the oxidation dye precursors are present in a quantity of from 0.05 to 5.0% by weight, based on the dyeing preparation as a whole.

6. Bis-(2,4-diaminophenyl)alkanes corresponding to general Formula (I) in Claim 1, in which R is an alkylene group containing three or four carbon atoms.

## Revendications

1. Teinture capillaire, contenant de premières fractions de colorants d'oxydation dans un support cosmétique, teinture caractérisée en ce qu'elle contient comme premières fractions de produits d'oxydation des alcanes bis-(2,4-diaminophényl) de formule générale:

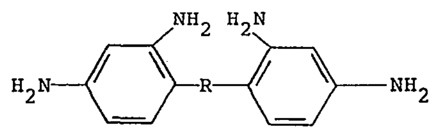

(I)

dans laquelle R est un groupe alkylène avec trois ou quatre atomes de carbone, ou bien leurs sels, comme copulants, ainsi que les substances de développement habituelles dans les teintures d'oxydation.

2. Teinture capillaire selon la revendication 1, caractérisée en ce que R est un groupe 1,3-propylène ou bien un groupe 1,4-butylène.

3. Teinture capillaire selon l'une des revendications 1 ou 2, caractérisée en ce que, comme substances de développement, elle contient des diamines aromatiques et hétérocycliques.

4. Teinture capillaire selon les revendications 1 à 3, caractérisée en ce qu'elle contient, en outre, une autre substance copulante connue et/ou des colorants montant directement.

5. Teinture capillaire selon les revendications 1 à 4, caractérisée en ce que la teneur en premières fractions de colorants d'oxydation est de 0,05 à 5,0% en poids de la teinture totale.

6. Alcanes bis-(2,4-diaminophényl) de formule générale (I) dans la revendication 1, dans laquelle R est un groupe alkylène avec 3 ou 4 atomes de carbone.